# EUROPEAN PATENT SPECIFICATION

(11) **EP 0 977 517 B1**
(45) Date of publication and mention of the grant of the patent: **06.07.2005**
(21) Application number: 98914342.5
(22) Date of filing: 31.03.1998
(51) Int. Cl.: A61B 19/08, A61F 13/15

(54) **ABSORBENT PAD FOR USE WITH SURGICAL DRAPES**
SAUGFÄHIGES KISSEN ZUR VERWENDUNG MIT EINEM CHIRURGISCHEN ABDECKTUCH.
CARRE ABSORBANT S'UTILISANT AVEC DES CHAMPS OPERATOIRES

(30) Priority: 15.04.1997 GB 9707559
(43) Date of publication of application: 09.02.2000
(73) Proprietor: MINNESOTA MINING AND MANUFACTURING COMPANY, St. Paul, Minnesota 55133-3427 (US)
(72) Inventor: SLOOTJES, Karin, T., M., Saint Paul, MN 55133-3427 (US)
(74) Representative: Aleandri-Hachgenei, Lorraine E.
(86) International application number: PCT/US1998/006185
(87) International publication number: WO 1998/046159

(56) References cited:
- EP-A- 0 483 816
- WO-A-96/01594
- US-A- 3 916 887
- US-A- 4 067 327
- US-A- 4 711 236
- US-A- 5 078 154
- US-A- 5 345 946
- US-A- 5 394 891

## Description

The present invention relates to drapes for use in covering the body of a patient during a surgical procedure and, more especially, to an absorbent pad for use with surgical drapes and to a method of using the absorbent pad.

The use of surgical drapes in operating theatres is well established. The drapes are employed to cover, and to separate, the non-sterile part of a patient's body from the sterile environment of the operating theatre and the operating theatre staff. Drapes are available in many different shapes and constructions, depending on the nature of the surgical procedure that is being carried out, and the manner in which the drapes are intended to be used.

To provide an effective barrier and prevent cross-contamination, the draping system used during a surgical procedure should provide a fluid-impervious layer which will prevent fluids generated during the procedure coming into contact with the patient's skin. It is also desirable that a draping system should be capable of absorbing at least some of the fluid generated during a surgical procedure although, when the presence of large amounts of fluid is expected, suitably-adapted drapes comprising fluid collection pockets or pouches can be used as described, for example, in US-A-4 616 642 and US-A-5 038 798.

Disposable surgical drapes, intended for a single use only, are being used increasingly to overcome problems associated with the sterilization of re-usable drape materials. Disposable drapes are supplied, already sterilized, in sterile packaging and are disposed of after use. They are frequently provided with adhesive strips which can be used to hold the drapes in position on the patient's body.

One form of disposable drape comprises a layer of a fluid impervious material (for example, a plastics film) which, in use, is placed closest to the patient's body and to which is laminated a fluid absorbent layer (for example, a non-woven material). A disadvantage of that type of construction is that the absorbent layer may not be absorbent enough to take up the fluid generated during a surgical procedure with the result that fluid may spill onto the floor of the operating theatre and present a hazard to the staff in the operating theatre. Another form of disposable drape comprises a non-woven material which may be chemically-treated to repel fluids and which, in a smaller area intended to be located adjacent the site of the surgical procedure, is provided with an integral absorbent pad. The absorbent pad may comprise, on the upper side of the pad, a layer of absorbent material and, on the lower side of the pad, a layer of fluid impervious material which, during manufacture, is permanently secured to the drape in a particular location by an adhesive. A disadvantage of constructions of that type is the lack of choice provided to operating theatre staff in the use and location of the absorbent pad.

The use on a surgical sheet, of adhesive strips which permit the attachment of additional drapes or sheets is described in US-A-3 916 887. In that case, the location of the adhesive strips determines the points at which the additional drapes or sheets can be attached.

Despite the advantages of disposable drapes, there are circumstances in which re-usable drapes, which should be laundered and then sterilized between each use, are more appropriate. Re-usable drapes are frequently formed of absorbent woven cotton or linen and often do not provide an effective fluid impervious barrier. In an attempt to overcome that problem, US-A-5 078 154 describes a drape system comprising a re-usable woven drape which has been treated to make it water-repellent and, in combination therewith, at least one re-usable woven towel which has been treated to make it absorbent and which, in use, is positioned on the water-repellant drape adjacent the operation site. It is disclosed that alternatively means may be provided for releasable attachment of the towels to drapes during use, for example press-stad fasteners or hook-and-loop type fasteners or water soluble double-sided adhesive tape or special fixers already in hospital use; or absorbent towels may be permanently attached to the drape sheets by means of an adhesive which will withstand the rigours of use, washing and sterilization.

US-A- 5 538 012 describes the use, in combination with a re-usable drape, of an impermeable disposable drape which surrounds the operation site and is secured to the re-usable drape by an adhesive.

The present invention is concerned with enabling the usefulness and applicability of draping systems to be increased in a comparatively simple manner.

The present invention provides an absorbent pad for use with surgical drapes, the pad being packaged and comprising, on one side, a layer of absorbent material and, on the other side, attachment means whereby the pad can be re-positionably attached directly to the outer surface of a surgical drape in any desired position, where in said attachment means comprises an adhesive, said adhesive being selected to allow the pad to be securely adhered to the outer surface of the drape material, to be removed without damaging the drape material and to be securely re-adhered, at least once, to the drape material.

In a preferred embodiment the absorbent pad for use with surgical drapes comprises a layer of absorbent material having an absorptive capacity (as hereinafter defined) of at least 500%.

In another preferred embodiment the absorbent pad for use with surgical drapes comprises, on one side, a layer of absorbent material having an absorptive capacity (as hereinafter defined) of at least 500% and, on the other side, the adhesive whereby the pad can be repositionably attached to the outer surface of a surgical drape in any desired position, the adhesive being covered by a layer of removable protective material.

An absorbent pad in accordance with the invention is typically, but not necessarily rectangular, and the adhesive is in the form of a strip and is located adjacent one edge of the pad.

The absorbent material of the pad may comprise a non-woven cellulosic or cellulosic blend material and may be a dry-laid non-woven material.

An absorbent pad in accordance with the invention may include a fluid impervious layer, for example a plastics film, between the absorbent material and the adhesive. The absorbent material may be thermally-bonded to the fluid impervious layer.

The term "non-woven" as used herein means a material that is produced differently from a fabric made by weaving or knitting and, more specifically, means a material that is produced by bonding and/or interlocking fibres, accomplished by mechanical, chemical or solvent means and combinations thereof.

By way of example only, embodiments of the invention will be described with reference to the accompanying drawings, which for clarity purposes do not show the packaging:
Fig. 1 is a plan view, from above, of an absorbent pad in accordance with the present invention;
Fig. 2 is a cross-section on the line II-II of Fig. 1, partly broken away, in which the thicknesses of the various layers of the pad are greatly exaggerated for clarity;
Fig. 3 is a diagrammatic plan view of a standard drape arrangement; and
Fig. 4 shows the same drape arrangement with the addition of two absorbent pads in accordance with the present invention.

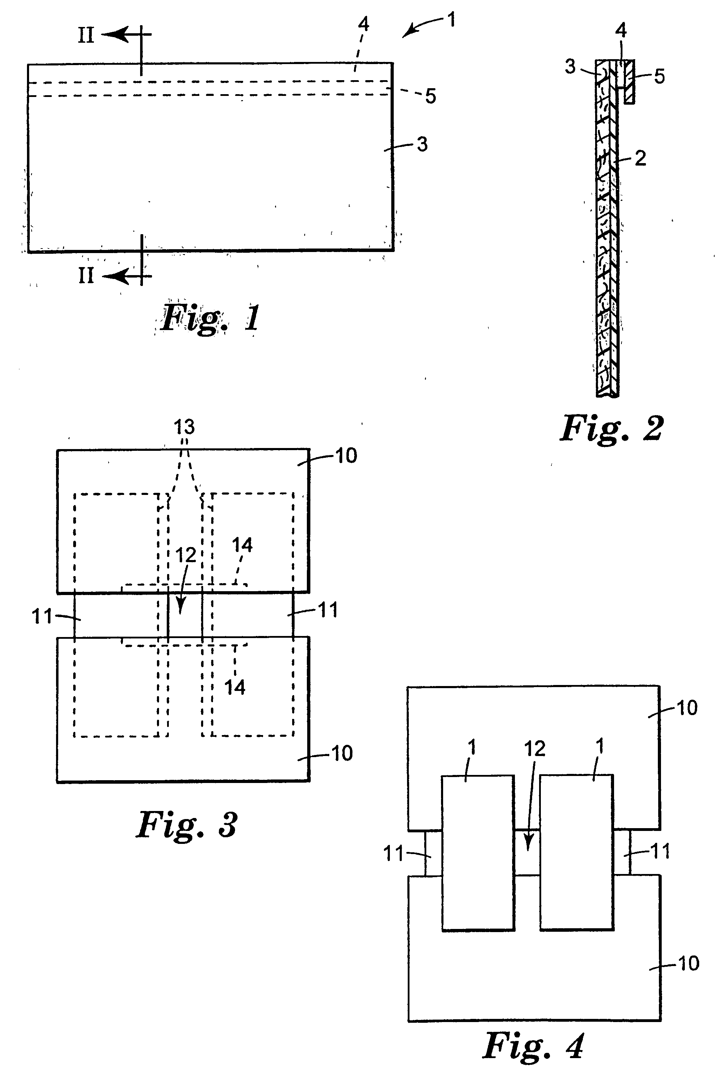

The absorbent pad 1 shown in Figs. 1 and 2 is generally rectangular in shape and comprises a backing layer 2 (not visible in Fig. 1) of a fluid impervious material, a layer 3 of absorbent material over the whole of the upper surface of the backing layer, and a strip of adhesive 4 along one of the longer edges of the back surface of the backing layer. The adhesive 4 is protected by a release liner 5.

The backing layer 2 comprises any suitable fluid impervious material, for example a plastics film or microporous film, or a closed-cell foam. In one embodiment, the backing layer is a polyethylene film having a thickness of 12 µm but it could also, for example, be formed from polypropylene, nylon, polyester, urethane, ethyl methylacrylate, or spun-bonded olefin and could have a thickness within the range 3 to 2500 µm. Generally, any fluid impervious material suitable for use in or with surgical drapes, and especially any plastics film material suitable for use in or with surgical drapes, could be used as the backing layer 2. However, other materials may also be suitable since the primary function of the backing layer 2, as will be apparent from the description below, is to form a liquid barrier rather than to impart strength to the pad 1.

The absorbent layer 3 is a non-woven material, for example a dry-laid cellulosic material. In one embodiment, the layer 3 is a dry-laid cellulose/vinyl acetate material available under the trade designation "FG403" from Walkisoft of Mt. Holly, North Carolina, U.S.A. In that embodiment, the layer 3 is thermally-bonded to the backing layer 2. Suitable alternative materials for the layer 3 include polyethylene and polypropylene blown microfibre materials and paper, and also non-linting super absorbent materials. Alternatively, the absorbent layer 3 may be a woven material (formed, for example, of cotton, linen or cellulose) or it may be a foamed material (for example, a urethane foam). The layer 3 may be formed from a material which is naturally absorbent or which has been treated to render it absorbent. Generally, as will be apparent from the description below, the material for the layer 3 is selected for its absorptive capacity rather than for its inherent strength and, from that point of view, when a non-woven material is used, a cellulosic material is preferred over, for example, a spun-bond material. The layer 3 preferably has an absorptive capacity of at least 500% and preferably at least 600% measured as described in ISO 9073-6 (Test methods for non-wovens-absorption), paragraph 5 (Liquid absorption capacity), using water as the test liquid. Layer 3 preferably also has an absorbency time of less than 6 seconds measured as defined in ISO 9073-6, paragraph 4 (Liquid Absorbency Time), using water as the test liquid. The absorbent layer 3 can be bonded to the upper surface of the backing layer 2 in any suitable way including, for example, by an adhesive.

The adhesive 4, as described below, allows the absorbent pad 1 to be adhered to the upper surface of surgical drape material in a secure manner and to be subsequently removed without damaging the drape material. The adhesive 4 is a repositionable adhesive and allows the pad 1 to be re-adhered to the drape material, again in a secure manner. The adhesive 4 is selected having regard to the surgical drape material with which the absorbent pad is intended to be used, and can advantageously be provided by laminating an adhesive-coated tape along one edge of the backing layer 2. For use with drape material having, as the upper surface, a non-woven polyester (or polyester-containing material), the adhesive 4 may be an acrylate adhesive and can conveniently and effectively be provided by double-coated medical tape available, under the trade designation "1509", from Minnesota Mining and Manufacturing Company of St. Paul, Minnesota, U.S.A. However, other forms of adhesive can be used and can be applied to the backing layer 2 in any suitable manner (including, for example, being sprayed, coated or laminated onto the backing layer).

The adhesive 4 is protected, as is conventional, by a release liner 5 (typically a release-coated paper) which is removed before the absorbent pad 1 is used.

The absorbent pad 1 can be of any suitable size but the sides of the pad typically have a length within the range 10 to 200 cm, and more specifically within the range 30 to 100 cm. Particularly appropriate sizes for the absorbent pad are 38 x 38 cm and 50 x 70 cm. The adhesive strip 4 is typically about 5 cm wide and the release liner 5 is slightly wider to provide a fingerlift by which the liner can be removed from the pad.

Although the absorbent pad 1 is described above as having two main layers only (i.e. the backing layer 2 and the absorbent layer 3), other layers could be included provided that the absorbent layer 3 is present at the upper surface of the pad and the adhesive strip 4 is present on the lower surface of the pad. For example, as mentioned above, there may be a layer of adhesive between the absorbent layer 3 and the backing layer 2. Alternatively, the absorbent layer 3 and/or the backing layer 2 could be multi-layer materials The absorbent layer 3 could, for example, comprise an upper layer selected to have a short absorbency time and a lower layer selected to have a high absorptive capacity. Moreover, although the inclusion in the pad 1 of the fluid impervious backing layer 2 is advantageous, it is not essential and in some cases the backing layer can be omitted.

Absorbent pads as described above are intended to be used in combination with surgical drapes to absorb fluids generated during the process of a surgical operation. They are of particular use, as will be described below, in combination with surgical drapes which are capable of absorbing a certain amount of fluid but which, in certain circumstances, do not have as high an absorptive capacity as might be desired. They are also of use in combination with drapes which have no absorptive capacity at all, for example because they have been treated to render them fluid repellent. The pads are supplied to the end user in some form of package, more particularly, a sterile package. They could, for example, be packaged as individual items or as part of a set of drapes.

Fig. 3 illustrates one particular drape arrangement with which absorbent pads 1 of the type shown in Fig. 1 can be used. The drape arrangement shown diagrammatically in Fig. 3 is a conventional disposable "four-towel" draping system comprising two large rectangular drapes 10 and two side towels 11. When in use in an operating theatre, the large drapes 10 are used to shield the upper and lower parts, respectively, of the body of a patient on an operating table and the side towels 11 are positioned at the sides, producing the arrangement as shown in Fig. 3 and defining a rectangular operation area 12 on the patient's body. The side towels 11, which are generally smaller than the drapes 10, each have an adhesive strip 13 on the lower surface along one long edge and are located on the patient, by means of these adhesive strips, before the drapes 10 are placed in position. Each of the drapes 10 also has an adhesive strip 14 on the lower surface, at least along the central region of one long edge, and are attached by the adhesive strip to the patient and to the upper surfaces of the side towels 11.

The drapes 10 and side towels 11 may be formed from a laminate material comprising at least a fluid impervious layer and, on the upper surface, a fluid absorbent layer. The fluid impervious layer is typically a plastics film, for example a polyethylene or a polypropylene film, and the absorbent layer may be a non-woven material, for example a polypropylene spun-bond material. The laminate may, however, also include other layers, for example a further layer of non-woven material between the upper absorbent layer and the fluid impermeable layer and/or a layer of material on the lower surface of the fluid impermeable layer.

Alternatively, the drapes 10 and side towels 11 may comprise a fluid repellent material which, in the region intended to lie adjacent the operation area 12 (Fig. 3) is provided with an integral absorbent area. The fluid repellent material may, for example, comprise a non-woven material which has been chemically treated to make it repellent to fluids. The integral absorbent area may comprise a pad of absorbent material with a fluid impermeable backing which is permanently bonded to the non-woven drape material with the absorbent material uppermost.

The conventional draping system illustrated in Fig. 3 is generally adequate in most cases in which it is employed. Disposable drapes 10 and side towels 11 generally are constructed to provide a fluid-impervious layer which will prevent fluids generated during a surgical procedure coming into contact with the patient's body, and they thus serve to separate the non-sterile part of the patient's body from the sterile environment of the operating theatre and the operating theatre staff. Disposable drapes 10 and side towels 11 are generally also capable of absorbing fluid generated during a surgical procedure, either because the whole upper surface of the drape/towel comprises a material with a certain absorptive capacity or because they have an integral absorptive area immediately adjacent the operation area 12.

In certain circumstances, however, the amount of fluid may exceed the absorptive capacity of conventional drapes/towels, and in that case a more specialized type of drape incorporating a fluid collection pouch or pocket is ideally employed. When that is not possible, it is common practice to cover soiled drapes with fresh ones and to repeat that as often as necessary during the course of an operation.

As a further alternative, in accordance with the present invention, one or more of the absorbent pads 1 described above can be attached, by the adhesive strip 4, to the drapes and towels 10, 11 of Fig. 3 adjacent the operation area 12. The pad(s) can be attached in any appropriate location where an increased absorptive capacity is required and can remain in place for as long as required and, if necessary, be re-positioned. Alternatively, if a pad becomes soiled, it can be removed and replaced by a fresh one. Fig. 4 shows the same drape arrangement as Fig. 3, to which two absorbent pads 1 have been added at the sides of the operation area 12 immediately above the side towels 11. It will be appreciated, however, that any number of pads 1 could be employed and that the pad(s) could be attached in any location adjacent the operation area 12.

When the absorbent pads 1 are used as shown in Fig. 4, they will hang in a generally vertical position at the sides of the patient, in which case the absorbency time of the absorbent material on the upper surface of the pads is of greater importance (and is preferably shorter) than when the pads are used in a generally horizontal position.

Because the adhesive strip 4 on the pad 1 is located exactly along the top edge of the pad, the formation of an undesirable "pocket" in which fluid could collect between the pad and the drape surface underneath is avoided. Depending on the circumstances in which the pad is used, this could be less important if the pad 1 has an absorbent material on its lower surface instead of the fluid impervious material 2.

It will be appreciated that when a pad 1 has been attached to a clean drape surface and is subsequently removed, either during the course of an operation or at the end, the drape surface immediately underneath the pad will be unsoiled. If pads are placed around the whole operation area at the beginning of an operation, a clean area can be obtained when required (for example, when an incision is being closed) simply by removing the pads.

Because the absorbent pad 1 is used in combination with other surgical drapes and does not, in itself, provide the barrier between the body of the patient and the sterile environment of the operating theatre, the pad does not need to have the same degree of strength and tear resistance that would be required of a surgical drape.

As mentioned above, the adhesive strip 4 on the absorbent pad 1 is selected to enable the pad to be securely attached to drape material and to be removable without damaging the drape material. The adhesive strip 4 advantageously also allows the pad to be re-positioned, at least once, on the drape material. A test carried out to assess the adhesion of the adhesive tape "1509" (mentioned above) on samples of various drape materials has indicated its suitability for use as the adhesive strip 4.

The test procedure that was used was as follows:
(i) A sample of drape material measuring 40 x 150 mm was placed, upper surface uppermost, on a flat surface.
(ii) A strip of the "1509" tape, measuring 25.4 x 152.4 mm, was positioned, adhesive side down, on the sample such that the strip was parallel to, and centred down the length of the sample (referred to below as the 1st application of the tape).
(iii) The tape was pressed onto the sample with a 5 Kg roller moved at a speed of approximately 50 mm/sec.
(iv) The tape and the sample were each clamped in a respective jaw of a tensile tester (a Hounsfield type H10KM), allowing the tape/sample assembly to hang freely. The machine settings were as follows: force recording range. 10 N; extension recording range, 300 mm.
(v) The jaws of the tensile tester were moved apart at a speed of 304.8 mm/min. and the force required to peel the tape from the sample was recorded. The measurements were made at a temperature of 23°C and a relative humidity of 50%.
(vi) When possible, steps (i) to (v) were repeated, using the same strip of tape and a fresh sample cut from the same lot of drape material (referred to below as the 2nd application of the tape).
(vii) When possible, step (vi) was repeated (referred to below as the 3rd application of the tape).

The drape materials that were used for the above-described test procedure were as follows:

| **Sample** | **Trade designation** | **Supplier** |
|---|---|---|
| 1 | Klinidrape, type 699040 | Mölnlycke Healthcare AB of Mölnlycke, Sweden |
| 2 | Steri Drape 9030 | Minnesota Mining and Manufacturing Company of St. Paul, Minnesota. USA |
| 3 | Raucodrape, type RDLS70S | Rauscher & Co. GmbH of Vienna, Austria |
| 4 | Barrier Drape, Sontara, type MPG001 | Johnson & Johnson of New Brunswick, New Jersey, USA |

The test results are summarized in the following Table (the delamination force in each case being the mean value obtained from measurements on eight samples cut from one lot of drape material):

| **Sample** | **Delamination force (N/2.54 cm)** | | |
|---|---|---|---|
| | **1st application** | **2nd application** | **3rd application** |
| 1 | 2.866 | * | * |
| 2 | 2.451 | 2.488 | 2.196 |
| 3 | 2.303 | 2.03 | 1.855 |
| 4 | 2.685 | 2.141 | 1.145 |

| | | | |
|---|---|---|---|
| * indicates that only one application was possible because the tape could not be removed without damaging the sample. | | | |

The test results indicated that the adhesive tape "1509" adhered acceptably to all of the samples of drape materials used and, except for one material, could be removed without damaging the material and re-applied twice, again with acceptable results. It was also observed that the same adhesive tape adhered to, and could be re-positioned on, non-treated woven cotton drapes.

Although the above description refers to the absorbent pad of Fig. 1 being used in combination with disposable drapes, it could also be used in combination with re-usable drapes when increased absorptive capacity is required. In that case, the effect of the pad in keeping the drapes clean immediately adjacent the operation area 12 could be particularly advantageous when the drapes are not entirely fluid impervious.

It will be appreciated that use of absorbent pads as described above with reference to Figs. 1 and 2 is not restricted to four-towel draping systems as illustrated in Fig. 3. The pads could be used with many other draping systems including, for example, split sheet drapes, aperture drapes, perineal area drapes and extremity drapes. It is also not essential for the pads to be rectangular in shape: when used with a split sheet drape, for example, a pad could be split in the same way as the drape.

## Claims

1. An absorbent pad for use in combination with surgical drapes, the pad being packaged and comprising, on one side, a layer of absorbent material and, on the other side, attachment means whereby the pad can be re-positionably attached directly to the outer surface of a surgical drape in any desired position, wherein said attachment means comprises an adhesive, said adhesive being selected to allow the pad to be securely adhered to the outer surface of the drape material, to be removed without damaging the drape material and to be securely re-adhered, at least once, to the drape material.

2. An absorbent pad as claimed in claim 1, in which the pad is contained in a sterile package.

3. An absorbent pad as claimed in claim 1 or claim 2, in which the layer of absorbent material has an absorptive capacity of at least 500% as measured in accordance with ISO 9073-6, paragraph 5 with water as the test liquid.

4. An absorbent pad as claimed in any one of the preceding claims, in which the adhesive is covered by a layer of removable protective material.

5. An absorbent pad as claimed in any one of the preceding claims, in which the adhesive is in the form of tape, which is laminated to the pad.

6. An absorbent pad as claimed in any one of the preceding claims, in which the absorbent pad is rectangular and the adhesive is in the form of a strip and is located adjacent to one edge of the pad.

7. An absorbent pad as claimed in any one of preceding claims, including a layer of fluid impervious matenal between the absorbent material and the adhesive.

8. An absorbent pad as claimed in any one of the preceding claims, in which the absorbent material comprises a non-woven material.

## Patentansprüche

1. Absorbierende Auflage zur Verwendung in Kombination mit chirurgischen Abdecktüchern, wobei die Auflage verpackt ist und auf einer Seite eine Schicht aus absorbierendem Material und auf der anderen Seite Anbringungsmittel umfasst, wodurch die Auflage in jeder gewünschten Position erneut positionierbar direkt an der Außenfläche eines chirurgischen Abdecktuchs anbringbar ist, wobei das Anbringungsmittel einen Klebstoff umfasst, wobei der Klebstoff derart ausgewählt ist, dass die Auflage sicher an die Außenfläche des Abdeckmaterials geklebt werden kann, ohne Beschädigung des Abdeckmaterials entfernt werden kann und mindestens einmal erneut sicher an das Abdeckmaterial geklebt werden kann.

2. Absorbierende Auflage nach Anspruch 1, wobei die Auflage in einer sterilen Verpackung aufgenommen ist.

3. Absorbierende Auflage nach Anspruch 1 oder Anspruch 2, wobei die Schicht aus absorbierendem Material eine Absorptionskapazität von mindestens 500 %aufweist, gemessen gemäß ISO 9073-6, Absatz 5, mit Wasser als Testflüssigkeit.

4. Absorbierende Auflage nach einem der vorhergehenden Ansprüche, wobei der Klebstoff durch eine Schicht eines entfernbaren schützenden Materials bedeckt ist.

5. Absorbierende Auflage nach einem der vorhergehenden Ansprüche, wobei der Klebstoff in Form eines Bandes ausgebildet ist, das auf die Auflage laminiert ist.

6. Absorbierende Auflage nach einem der vorhergehenden Ansprüche, wobei die absorbierende Auflage rechteckig ist und der Klebstoff in Form eines Steifens ausgebildet ist, der angrenzend an einen Rand der Auflage angeordnet ist.

7. Absorbierende Auflage nach einem der vorhergehenden Ansprüche, welche eine Schicht aus fluidundurchlässigem Material zwischen dem absorbierenden Material und dem Klebstoff umfasst.

8. Absorbierende Auflage nach einem der vorhergehenden Ansprüche, wobei das absorbierende Material ein nichtgewebtes Material umfasst.

## Revendications

1. Carré absorbant destiné à une utilisation en combinaison avec des champs opératoires, le carré étant emballé et comprenant, sur un côté, une couche de matériau absorbant et, sur l'autre côté, un moyen de fixation par lequel le carré peut être fixé de façon repositionnable directement à la surface externe d'un champ opératoire, dans n'importe quelle position souhaitée, dans lequel ledit moyen de fixation comprend un adhésif, ledit adhésif étant sélectionné pour permettre au carré d'être solidement collé sur la surface externe du matériau constituant le champ, d'être décollé sans abîmer le matériau constituant le champ, et d'être solidement collé de nouveau, au moins une fois, sur le matériau constituant le champ.

2. Carré absorbant selon la revendication 1, dans lequel le carré est contenu dans un emballage stérile.

3. Carré absorbant selon la revendication 1 ou la revendication 2, dans lequel la couche de matériau absorbant a une capacité d'absorption d'au moins 500 %, mesurée selon ISO 9073-6, paragraphe 5, l'eau étant utilisée comme liquide d'essai.

4. Carré absorbant selon l'une quelconque des revendications précédentes, dans lequel l'adhésif est recouvert d'une couche de matériau protecteur amovible.

5. Carré absorbant selon l'une quelconque des revendications précédentes, dans lequel l'adhésif est sous la forme d'un ruban, qui est appliqué par stratification sur le carré.

6. Carré absorbant selon l'une quelconque des revendications précédentes, dans lequel le carré absorbant est rectangulaire et l'adhésif est sous la forme d'une bande et positionné de façon adjacente à un bord du carré.

7. Carré absorbant selon l'une quelconque des revendications précédentes, comprenant une couche de matériau imperméable aux fluides entre le matériau absorbant et l'adhésif.

8. Carré absorbant selon l'une quelconque des revendications précédentes, dans lequel le matériau absorbant comprend un matériau non tissé.
